# EUROPEAN PATENT SPECIFICATION

(11) **EP 1 086 695 B1**
(45) Date of publication and mention of the grant of the patent: **22.12.2004**
(21) Application number: 99118619.8
(22) Date of filing: 21.09.1999
(51) Int. Cl.: A61K 31/475, A61P 31/12, A61P 31/16, A61P 31/22

(54) **Antiviral compositions comprising yohimbine as active principle**
Antivirale Zusammensetzungen enthaltend Yohimbin als therapeutischen Wirkstoff
Compositions antivirales comprenant de la yohimbine en tant que principe actif

(43) Date of publication of application: 28.03.2001
(73) Proprietor: Leone, Enzo, 20124 Milano (IT)
(72) Inventor: Leone, Enzo, 20124 Milano (IT)
(74) Representative: Gervasi, Gemma, Dr.

(56) References cited:
- WO-A-98/29101
- FR-A- 2 765 483
- US-A- 5 109 007
- ALARCON, BALBINO ET AL: "Screening for new compounds with antiherpes activity" ANTIVIRAL RES., 1984, 4, 231-44, XP000885848
- KUSUMOTO, INES TOMOCO ET AL: "Effects of flavonoids and alkaloids on reverse transcriptase" SHOYAKUGAKU ZASSHI, 1991, 45, 240-54, XP000579880

## Description

### Scope of the invention

The present invention refers to the use of yohimbine as acitve principle for the preparation of therapeutic compositions with antiviral activity. The invention also refers to the use of pharmacologically acceptable yohimbine salts for the preparation of pharmaceutical compositions having antiviral activity.

### Prior art

Yohimbine (C₂₁H₂₆N₂O₃, methyl 17-alpha-hydroxy-20-alpha-yohimban-16-beta-carboxylate, m.p. 243-244°C) is an indole alkylamine alkaloid contained in the bark of Pausinystalia johimbe, a tree belonging to the Rubiaceae family, in the bark of Corynanthe johimbe and Pseudocinchona johimbe, as well as in the root of Rauwolfia serpentina, from the alcoholic extract of which it has been isolated together with other alkaloids similar to it as regards chemical composition (Chatterjee, Experientia, Vol. X/6 246-247 (1954). The use of yohimbine as a drug has been known (R. Benigni et al., PIANTE MEDICINALI - Chimica, Farmacologia, Terapia, Vol. II - Inverni della Beffa, Milano 1964) since last century both as a vasodilator and, at high doses, as a vasoconstrictor. Its activity on the vascular system has been amply described, and the specificity of action of yohimbine, its pharmacological kinetics, as well as its selectivity towards alpha2-adrenergic receptors, make it the ideal instrument in pharmacological dissections for the study of animal and human physiology, as well as for the study of the mechanism of action of other active principles, in particular anaesthetics and antihypertensives. It is used in the treatment of impotence on an organic basis. Yohimbine hydrochloride has moreover been successfully employed in the treatment of the sequelae of surgical operations on the abdomen (intestinal atony).

WO 98/29101 discloses that glutathione or a glutathione derivative has potential for the treatment of impotence and may also be co-administered with yohimbine, providing synergistic effect.

In a large study, I.T. Kusumoto et al. (Shoyakugaku Zasshi, 1991, 45/3 240-254) tested flavonoids and alkaloids on reverse transcriptase and concluded that, of the alkaloids tested, isoquinoline alkaloids and other alkaloids including indol (among which yohimbine), steroidal, quinolizilidine, pyridine and imidazole alkaloids containing no quaternary nitrogen did not show any significant reverse trancriptase activity.

In another study, B.Alarcon et al. (Antiviral Research 1984, 4, 231-143) carried out a screening of a number of compounds for antiherpes activity and found that yohimbine hydrocloride proved inactive against HSV-1 in their screening system.

So far no effects of yohimbine as antiviral agent have been reported.

At present, antiviral therapy resorts to highly toxic products, which are frequently poorly tolerated, are inactive on the evolution of the infection, and are very costly.

### Summary of the invention

It has now been found, unexpectedly with respect to the state of the art, that yohimbine, in all its isomeric forms, and in particular alpha- yohimbine, possesses effective antiviral properties, in particular antiviral properties in regard to herpes virus 1 and 2, in its multiform cutaneous and keratoconjunctival manifestations, as well as in regard to influenza and para-influenza syndromes.

An object of the present invention is therefore the use of yohimbine, its pharmacologically acceptable salts, in particular yohimbine hydrochloride, and corresponding mixtures, to produce pharmaceutical compositions having antiviral activity. In particular, it has been found, and it constitutes an object of the present invention, that it is possible to produce pharmaceutical compositions comprising yohimbine and/or its pharmacologically acceptable salts which are active in regard to herpes virus 1 and 2, influenza virus and para-influenza virus.

Further objects will be evident from the following detailed description of the invention.

### Detailed description of invention

The pharmaceutical compositions having antiviral activity, comprising as active principle yohimbine and/or its pharmacologically acceptable salts, in particular yohimbine hydrochloride, may contain other active principles having activity different from, and complementary to, the activity of the said compositions, for the treatment of given conditions (for example, Eleutherococcus, Hypericum and their derivatives).

The compositions may contain yohimbine and/or its salts as sole active principle, mixed with appropriate excipients and/or diluents. Suitable excipients and diluents are, for example, magnesium stearate, rice starch, and talcum.

The antiviral activity of yohimbine has been assayed using its hydrochloride for reasons of solubility and stability of the molecule, and hence convenience of use, but it is to be understood as identically manifested by free yohimbine and by the other salts of yohimbine. It has been found that the doses at which yohimbine is effective as antiviral agent are 3-10 mg/day, expressed as free base. The preferred dosage is 5 mg/day from the onset of the first signs of the viral infection up to its cure. In the case of long courses of treatment, it is preferable to follow a scheme of two- or three-month cycles with intervals of rest of 10-15 days. Higher doses than the range indicated may result in a failure of the curative effect, this being due to the peculiarity of yohimbine, observed on various organs and apparatus, namely of stimulating an action (for example, splenocontraction) at a certain dose level, and the opposite action (splenodilation) at higher dose levels. Doses below the limit indicated may be therapeutically ineffective.

In the literature there are no cases reported of intoxication resulting from yohimbine (either yohimbine alone or its salts) either due to oversensitivity to the product or to ingestion of high doses of the substance. Yohimbine-based drugs are safe and well tolerated at the indicated doses. Compositions based on yohimbine and/or its salts having antiviral activity are substantially free from side effects at the doses used, this representing a decided advantage as compared to the antiviral drugs currently in use. In fact, at present antiviral therapy resorts to highly hepatotoxic products, which are often poorly tolerated and are not active on the evolution of the infection. To mention just a few: amantadine (Mantadan), rimantadine, and acyclovir (Zovirax).

The compositions according to the invention may be suited for topical use, for example in the treatment of skin affections, or may be formulated as solutions for oral or parenteral administration. In the latter case, yohimbine salts are preferred, and in particular yohimbine hydrochloride.

The preparations for oral use are diluted so as to obtain from 0.01 to 0.1 g/l of yohimbine.

The phials for parenteral use contain from 3 to 5 mg of active principle. Given below are the results, which are summarized for reasons of simplicity, of a number of clinical tests carried out using the compositions according to the invention.

The examples given are to be considered a mere illustration of the present invention.

### Example 1

### Treatment of herpes

In the course of 7 years, 30 patients suffering from herpes virus 1 or 2 were treated, the said patients still being currently under supervision.

The treatment involved the use of 5 mg/day of yohimbine HCI until resolution of the dermatological affections.

In all cases, the resolution occurred in a period of time ranging from 1 to 6 months of treatment, with a considerable reduction in the number of cases of relapse; in just some cases, relapse occurred only after quite a few years, and was of minor proportions.

### Example 2

### Treatment of herpetic keratitis

Ten (10) subjects affected by herpetic keratoconjunctivitis, who had undergone classic treatment (acyclovir) without any benefit in terms of recidivation, this being both painful and of long duration, were treated with yohimbine HCI at the dose level of 5 mg/day. With this treatment, the cases of relapse were less frequent and of shorter duration, and also the pain involved became more tolerable in the periods when the affection again became more acute. These re-occurrences of acute episodes were, however, mastered with three-week cycles of treatment using the drug in question, with three-week rest intervals between one cycle and another. Treatment has lasted for several years now, without any problems of any sort arising.

### Example 3

### Treatment of influenza syndromes

All the forms of influenza treated, including those involving elderly patients and children, benefited from a 7-10 day treatment with yohimbine HCI at the dosage of 5 mg/day, leading to a complete resolution.

### Example 4

### Treatment of herpes progenitalis in a serious form

A 39-year-old woman, who had suffered for over twenty years from herpes progenitalis recidivating even once or twice a month, had been treated unsuccessfully with every specific drug (Zovirax, Idustatin) and had even tried non-official forms of therapy (homeopathy and phytotherapy) without any benefit. The patient took 5 mg/day of yohimbine HCI for more than one year with three-week rest intervals every three months, using a galenical preparation consisting of:

| | |
|---|---|
| yohimbine HCI | 5 mg |
| carboxymethyl cellulose | 50 mg |
| magnesium stearate | 10 mg |
| rice starch | 50 mg |
| talcum | 90 mg |
| | per tablet. |

This treatment did not reveal any side effect and within approximately six months led to the disappearance of the lesions and of the vesicular eruptions. A cyclic treatment was kept up for a further six months until the affection was deemed resolved.

Only one other slight episode of recurrence occurred five years later. Moreover, the patient gave birth to a child without problems of any sort arising. No other drugs were associated to the yohimbine-based therapy.

## Claims

1. The use of yohimbine, its pharmacologically acceptable salts, or their mixtures, for the preparation of pharmaceutical compositions having antiviral activity.

2. Use according to claim 1, wherein said pharmaceutically acceptable salt is the hydrocloride.

3. Use according to anyone of claims 1 and 2, wherein said yohimbine is in anyone of its isomeric forms.

4. Use according to claim 3, wherein said its isomeric form is alpha-yohimbine.

5. Use according to anyone of claims 1 to 4, wherein said antiviral activity is in regard to herpes virus 1 and 2, influenza virus and para-infuenza virus.

6. Use according to anyone of claims 1 to 5, wherein said antiviral activity is in regard to herpetic keratitis.

7. Use according to anyone of claims 1 to 6, wherein said pharmaceutical compositions are for administration of a yohimbine dose of from 3 to 10 mg/day.

8. Use according to anyone of claims 1 to 7, wherein said pharmaceutical compositions are for administration of a yohimbine dose of from 3 to 5 mg/day.

9. Use according to anyone of claims 1 to 8, wherein said pharmaceutical compositions are for topical administration or formulated as solutions for oral or parenteral administration.

10. Use according to anyone of claims 1 to 9, wherein in said pharmaceutical compositions said yohimbine is the sole active principle.

11. Use according to claim 10, wherein said pharmaceutical compositions are in form of tablets each containing 5 mg of yohimbine hydrocloride destined to the treatment of herpes progenitalis.

## Patentansprüche

1. Verwendung von Yohimbin, dessen pharmazeutisch annehmbaren Salze, oder ihre Mischungen, zur Herstellung von parmazeutischen Zubereitungen, die antivirale Wirkung haben.

2. Verwendung nach Anspruch 1, **dadurch gekennzeichnet, dass** der pharmazeutisch annehmbare Salz das Hydrochlorid ist.

3. Verwendung nach mindestens einem der Ansprüche 1 und 2, **dadurch gekennzeichnet, dass** das Yohimbin in eine von seinen isomeren Formen ist.

4. Verwendung nach Anspruch 3, **dadurch gekennzeichnet, dass** die isomere Form alpha-Yohimbin ist.

5. Verwendung nach einem der Ansprüche 1 bis 4, **dadurch gekennzeichnet, dass** die antivirale Wirkung gegen Herpes Virus, Influenza Virus,and Parainfluenza Virus ist.

6. Verwendung nach einem der Ansprüche 1 bis 5, **dadurch gekennzeichnet, dass** die antivirale Wirkung gegen Keratitis herpetica ist.

7. Verwendung nach einem der Ansprüche 1 bis 6, **dadurch gekennzeichnet, dass** diese parmazeutische Zubereitungen zur Verabreichung einer Yohimbindosierung von 3 bis 10 mg/Tag sind.

8. Verwendung nach einem der Ansprüche 1 bis 7, **dadurch gekennzeichnet, dass** diese parmazeutische Zubereitungen zur Verabreichung einer Yohimbindosierung von 3 bis 5 mg/Tag sind.

9. Verwendung nach mindestens einem der Ansprüche 1 bis 8, **dadurch gekennzeichnet, dass** diese parmazeutische Zubereitungen zur topischen Verabreichung oder als Lösungen zur oralen oder parenteralen Verabreichung formuliert sind.

10. Verwendung nach mindestens einem der Ansprüche 1 bis 9, **dadurch gekennzeichnet, dass** diese parmazeutische Zubereitungen Yohimbin als einziger Wirkstoff enthalten.

11. Verwendung nach Anspruch 10, **dadurch gekennzeichnet, dass** diese parmazeutische Zubereitungen in die Form von 5 mg Yohimbinhydrochlorids enthaltenden Tabletten zur Behandlung von Herpes progenitalis sind.

## Revendications

1. Utilisation de la yohimbine, ses sels pharmaceutiquement acceptables, ou leur mélanges, pour la préparation de compositions pharmaceutiques ayant activité antivirale.

2. Utilisation selon la revendication 1, **caractérisée en ce que** ledit sel pharmaceutiquement acceptable est le chlorhydrate.

3. Utilisation selon l'une quelconque des revendications 1 et 2, **caractérisée en ce que** ladite yohimbine est dans une quelconque de ses formes isomériques.

4. Utilisation selon la revendication 3, **caractérisée en ce que** ladite forme isomérique est l'alpha-yohimbine.

5. Utilisation selon l'une quelconque des revendications de 1 à 4, **caractérisée en ce que** ladite activité antivirale est sur le virus de l'herpès, le virus de l'influenza et le virus de la para-influenza.

6. Utilisation selon l'une quelconque des revendications de 1 à 5, **caractérisée en ce que** ladite activité antivirale est sur la kératite herpétique.

7. Utilisation selon l'une quelconque des revendications de 1 à 6, **caractérisée en ce que** lesdites compositions pharmaceutiques sont pour l'administration d'une dose de yohimbine de 3 à 10 mg/jour.

8. Utilisation selon l'une quelconque des revendications de 1 à 7, **caractérisée en ce que** lesdites compositions pharmaceutiques sont pour l'administration d'une dose de yohimbine de 3 à 5 mg/jour.

9. Utilisation selon l'une quelconque des revendications de 1 à 8, **caractérisée en ce que** lesdites compositions pharmaceutiques sont pour l'administration topique ou formulées comme solutions pour l'administration orale ou parentérale.

10. Utilisation selon l'une quelconque des revendications de 1 à 9, **caractérisée en ce que** dans lesdites compositions pharmaceutiques ladite yohimbine est le seul principe actif.

11. Utilisation selon la revendication 10, **caractérisée en ce que** lesdites compositions pharmaceutiques sont sous forme de comprimés chacun contenant 5 mg de chlorhydrate de yohimbine destinés au traitement de l'herpes progenitalis.
